# EUROPEAN PATENT APPLICATION

(11) **EP 2 578 245 A2**
(43) Date of publication of application: **10.04.2013**
(21) Application number: 12182662.2
(22) Date of filing: 31.08.2012
(51) Int. Cl.: A61L 27/04, A61L 27/34, A61L 31/02, A61L 31/10, A61L 27/54, A61L 31/16

(54) **Hydrogel coated magnesium medical implants**

(30) Priority: 01.09.2011 US 201161530117 P; 27.08.2012 US 201213595246
(71) Applicant: Covidien LP, Mansfield, MA 02048 (US)
(72) Inventor: Hodgkinson, Gerald, Guilford, CT Connecticut 06437 (US); Hadba, Ahmad Robert, Fort Worth, TX Texas 76109 (US)
(74) Representative: Maschio, Antonio

(57) **Abstract**

A surgical implant is provided which includes a body and a coating in contact with at least a portion of the body, the body including metallic magnesium, the coating including a hydrogel having an adhesion peptide contained therein. The adhesion peptide may be derived from an extracellular matrix protein and may be covalently bonded to the hydrogel. A method of making a surgical implant includes providing a magnesium based degradable implant body; applying and adhering a functionalized reactive silane based adhesion promoting layer to the implant body; providing a hydrogel monomeric solution having extracellular matrix adhesion peptides incorporated therein; and contacting the hydrogel monomeric solution with the adhesion promoting layer such that the hydrogel polymerizes and bonds to the adhesion promoting layer and encapsulates at least a portion of the implant.

## Description

### CROSS-REFERENCE TO RELATED APPLICATIONS

This application claims the benefit of and priority to U.S. Provisional Patent Application No. 61/530,117, filed September 1, 2011, the entire disclosure of which is incorporated by reference herein.

### BACKGROUND

### Technical Field

The present disclosure relates generally to magnesium medical implants. More particularly, the present disclosure relates to magnesium implants having hydrogel coatings which delay or prevent degradation of such implants and facilitate attachment of such implants to a target site.

### Description of Related Art

Magnesium and magnesium alloys have been processed into medical implants for use in animals and humans (referred to herein collectively as "magnesium implant(s)" or simply "implant(s)"). Magnesium implants degrade over time *in situ* and can advantageously be formulated to possess density and strength in load bearing applications that correspond to bone. Magnesium stents have also been formulated. However, in certain instances, faster than desirable degradation rates, hydrogen gas evolution and degradation products which increase local pH (alkalosis) have been problematic. Hydrogels have been used as a coating on magnesium implants to control rate of degradation and to reduce the risk of developing alkalosis. See, e.g., US Pat. Appln. Pub. Nos. 2010/0023112 and 2009/0240323. However, hydrogels frequently have a lubricious surface *in situ* which can result in difficulty in adhering an implant having a hydrogel coating to surrounding tissue and maintaining the position the implant.

There is a need for magnesium implants which have controlled or reduced rate of degradation, which do not cause local alkalosis, which promote cellular attachment and adhere to a surgical target site.

### SUMMARY

A surgical implant is provided which includes a body and a coating in contact with at least a portion of the body, the body including metallic magnesium, the coating including a hydrogel having an adhesion peptide contained therein. In some embodiments, the adhesion peptide may be derived from an extracellular matrix protein. In embodiments, the adhesion peptide is covalently bonded to the hydrogel. In embodiments the hydrogel may be polyethylene glycol, alginate, collagen and/or polyurethane.

A method of making a surgical implant includes providing a magnesium based degradable implant body; applying and adhering a functionalized reactive silane based adhesion promoting layer to the implant body; providing a hydrogel monomeric solution having extracellular matrix adhesion peptides incorporated therein; and contacting the hydrogel monomeric solution with the adhesion promoting layer such that the hydrogel polymerizes and bonds to the adhesion promoting layer and encapsulates at least a portion of the implant.

### BRIEF DESCRIPTION OF THE DRAWINGS

The accompanying drawings, which are incorporated in and constitute a part of this specification, illustrate aspects of the presently disclosed hydrogel coated magnesium implants, and together with a general description and the detailed description of the embodiments of the disclosed magnesium implants herein given, serve to explain certain principles of the disclosed magnesium implants.

FIG. 1 is a graph illustrating mass loss of magnesium alloy samples versus polyethylene glycol content of encapsulating hydrogel coatings.

FIG. 2 is a graph illustrating diffusion rates of fluoroceine (FITC) dye through hydrogel membranes of varying polyethylene glycol content.

### DETAILED DESCRIPTION

Magnesium implants herein include a body made of magnesium and/or a magnesium alloy and a hydrogel coating which incorporates one or more extracellular matrix adhesion peptides (ECMAPs). In embodiments, the hydrogel coating may partially or completely encapsulate the body. In some embodiments, the hydrogel is permeable to aqueous solutions. In some embodiments, the hydrogel coating is substantially impermeable to aqueous solutions. In some embodiments, the hydrogel coating is degradable. In some embodiments the hydrogel coating is non-degradable. Without wishing to be bound by any particular theory, it is believed that the coatings act to reduce degradation of the magnesium body by limiting aqueous solution exchange at the surface of the magnesium implant and by reducing diffusion of ions proximate to the implant surface that participate in normal magnesium degradation reactions. Such ions include Cl⁻, SO₄⁻ and OH⁻. By limiting diffusion of OH⁻ ions (which are normal products of magnesium degradation in aqueous solution) away from the implant, irritation from local alkalosis is reduced or eliminated. In addition, since magnesium degradation in aqueous solution halts at a pH greater than 12, sequestering OH- ions proximate to the surface of the implant body causes an increase in local pH at the body which reduces or halts magnesium degradation. In essence, continued release of OH- ions caused by magnesium degradation into a static environment created by the coating leads to a self-limiting chemical reaction. In addition, incorporation of acrylate groups into the hydrogel promotes reduction of free OH- ions by hydrolysis with acrylate groups.

The tendency for lubricious hydrogel surfaces to be relatively frictionless or slippery may be disadvantageous when an implant is intended to fixed in place and load bearing or if cellular attachment and/or in-growth of surrounding tissue is desirable. Incorporation of extracellular matrix adhesion peptides into the hydrogel promotes cellular attachment to and into the hydrogel coating, thus stabilizing the magnesium implant at a target site. Suitable extracellular matrix adhesion peptides are known in the art and include RGD, YIGSR, KQAGDV, REDV, PHSRN, IKVAV, PDGSR, LRGDN, LRE, IKLLI, GFOGER and VAPG.

In some embodiments, a hydrogel incorporating extracellular matrix adhesion peptides is non-degradable and permeable. Such non-degradable tissue in-growth inductive materials can act as a mechanical support for the implant as tissue grows into the coating and around the implant as the implant degrades. This provides a benefit over degradable materials in which implant loosening during degradation of either a magnesium core or a magnesium coating causes prolonged healing and/or potential failure of the implant. This would be especially advantageous for load bearing implants such as in orthopedics or for implants used in or near moving tissues such as in muscles or in joints. In some embodiments, a hydrogel incorporating extracellular matrix adhesion peptides is degradable and permeable which ultimately results in a degradable magnesium implant that can have a variable rate of degradation. The rate is initially slower while the degradable coating remains relatively intact and cellular attachments are formed. As the coating degrades, the underlying implant body is exposed to the aqueous solution in greater amounts with a consequent increase in diffusion and degradation of the implant.

Examples of suitable hydrogel materials include polyethylene glycol (PEG), alginate, urethane and cross-linked collagen. PEG may have linear or branched multiarm structures. For incorporation of extracellular matrix adhesion peptides, one or both of the two hydroxyl end groups of PEG can be converted to functional groups such as methyloxyl, carboxyl, amine, thiol, azide, vinyl sulfone, azide, acetylene and acrylate. The end groups may be the same or different which allows for a plethora of combinations of functional end group links to extracellular matrix adhesion peptides. Those skilled in the art are familiar with techniques for converting the end groups and coupling peptides thereto. See, e.g., Zhu, Biomaterials 31 (2010) 4639-4656. For example, a PEG hydrogel may be prepared by photopolymerization of PEG diacrylate (PEGDA). Acrylic acid may be copolymerized with PEGDA to provide carboxyl groups available for conjugation to amine groups of extracellular matrix adhesion peptides. Larger amounts of acrylic acid will allow for larger amounts of extracellular matrix adhesion peptides to be incorporated into the copolymer. To promote in-growth, it may be advantageous to incorporate extracellular matrix adhesion peptides throughout the three-dimensional structure of the hydrogel. Copolymerization of PEGDA with monoacrylated extracellular matrix adhesion peptides may be accomplished by functionalizing the N-terminal amines of the peptides with N-hydroxyl succinimide. Modification of PEG hydrogels by attachment of maleimide or thiol groups allows utilization of Michael-type addition to incorporate extracellular matrix adhesion peptides.

Urethane based hydrogels may be utilized with incorporated extracellular matrix adhesion peptides in accordance with the present disclosure. In embodiments, urethanes herein contain functional groups, such as carboxylic acid groups, which can be used as anchor sites for extracellular matrix adhesion peptide binding. For example, bioactive polyurethaneurea presenting YIGSR is synthesized by incorporating GGGYIGSRGGGK peptide sequences into the polymer backbone. See, Jun, et al. J Biomater. Sci. Polym. Ed. 2004;15(1):73-94. A biodegradable poly(ester-urethane)urea (PEUU) containing RGDS is synthesized from polycaprolactone and 1,4-diisocyanatobutane, with putrescine used as a chain extender. See, Guan et al. Engineering in Medicine and Biology, 2002. 24th Annual Conference and the Annual Fall Meeting of the Biomedical Engineering Society EMBS/BMES Conference, 2002. Proceedings of the Second Joint, Volume: 1, page(s): 761 - 762 vol. 1. Polyurethane scaffolds can be modified with radio frequency glow discharge followed by surface coupling of RGDS peptide. Id. Polyethylene glycol modified polyurethane (PU-PEG) may also be utilized in accordance with the present disclosure. Cell adhesive peptide Gly-Arg-Gly-Asp (GRGD) can be photochemically grafted to the surface of the hydrogel utilizing GRGD-N-Succinimidyl-6-[4'-azido-2'-nitrophenylamino]hexanoate (SANPAH) on a PU-PEG surface through adsorption and subsequent ultraviolet irradiation. See, Lin et al., Artif Organs. 2001 Aug;25(8):617-21.

Alginates may be covalently modified with extracellular matrix adhesion peptides by formation of an amide bond between the carboxylic acid groups on the alginate chain and amine groups on the cell adhesion molecule. Alginates may also be covalently modified with extracellular matrix adhesion peptides utilizing aqueous carbodiimide chemistry. See, e.g., Rowley et al., Biomaterials. 1999 Jan; 20(1):45-53 (covalent modification of alginate with GRGDY peptides using carbodiimide functional crosslinkers). Extracellular matrix adhesion peptides contain a terminal amine group for such bonding. The amide bond formation may be catalyzed by 1-ethyl-3-(3-dimethylaminopropyl)carbodiimide (EDC), which is a water soluble enzyme commonly used in peptide synthesis. EDC reacts with carboxylate moieties on the alginate backbone creating activated esters which are reactive towards amines. To reduce unfavorable side reactions, EDC may be used in conjunction with N-hydroxysuccinimide, N-hydroxysulfylsuccinimide or 1-hydroxybenxotriazole (HOBT) to facilitate amide bonding over competing reactions.

In general, hydrogels should be linked to extracellular matrix adhesion peptides by cross-linking procedures which preferably do not cause denaturing or misfolding of the extracellular matrix adhesion peptides. The terms "linked" or "conjugated" are used interchangeably herein and are intended to include any or all of the mechanisms known in the art for coupling a hydrogel to a extracellular matrix adhesion peptide. For example, any chemical or enzymatic linkage known to those with skill in the art is contemplated including those which result from photoactivation and the like. Homofunctional and heterobifunctional cross linkers are all suitable. Reactive groups which can be cross-linked with a cross-linker include primary amines, sulfhydryls, carbonyls, carbohydrates and carboxylic acids. For example, PEG may be covalently bound to amino acid residues via a reactive group. Reactive groups are those to which an activated PEG molecule may be bound (e. g., a free amino or carboxyl group). For example, N-terminal amino acid residues and lysine (K) residues have a free amino group and C-terminal amino acid residues have a free carboxyl group. Sulfhydryl groups (e.g., as found on cysteine residues) may also be used as a reactive group for attaching PEG. In addition, enzyme-assisted methods for introducing activated groups (e.g., hydrazide, aldehyde, and aromatic-amino groups) specifically at the C-terminus of a polypeptide may be utilized.

Cross-linkers are conventionally available with varying lengths of spacer arms or bridges. Cross-linkers suitable for reacting with primary amines include homobifunctional cross-linkers such as imidoesters and N-hydroxysuccinimidyl (NHS) esters. Examples of imidoester cross-linkers include dimethyladipimidate, dimethylpimelimidate, and dimethylsuberimidate. Examples of NHS-ester cross-linkers include disuccinimidyl glutamate, disucciniminidyl suberate and bis (sulfosuccinimidyl) suberate. Accessible amine groups present on the N-termini of peptides react with NHS-esters to form amides. NHS-ester cross-linking reactions can be conducted in phosphate, bicarbonate/carbonate, 4-(2-hydroxyethyl) piperazine-1-ethane sulfonic acid (HEPES), 3-(N-morpholino) propane sulfonic acid (MOPS) and borate buffers. Other buffers can be used if they do not contain primary amines. The reaction of NHS-esters with primary amines should be conducted at a pH of between about 7 and about 9 and a temperature between about 4°C and 30°C for about 30 minutes to about 2 hours. The concentration of NHS-ester cross-linker can vary from about 0.1 to about 10 mM. NHS-esters are either hydrophilic or hydrophobic. Hydrophilic NHS-esters are reacted in aqueous solutions although DMSO may be included to achieve greater solubility. Hydrophobic NHS-esters are dissolved in a water miscible organic solvent and then added to the aqueous reaction mixture

Sulfhydryl reactive cross-linkers include maleimides, alkyl halides, aryl halides and a-haloacyls which react with sulfhydryls to form thiol ether bonds and pyridyl disulfides which react with sulfhydryls to produce mixed disulfides. Sulfhydryl groups on peptides and proteins can be generated by techniques known to those with skill in the art, e.g., by reduction of disulfide bonds or addition by reaction with primary amines using 2-iminothiolane. Examples of maleimide cross-linkers include succinimidyl 4-{N-maleimido-methyl) cyclohexane-1-carboxylate and m-maleimidobenzoyl-N-hydroxysuccinimide ester. Examples of haloacetal cross-linkers include N-succinimidyl (4-iodoacetal) aminobenzoate and sulfosuccinimidyl (4-iodoacetal) aminobenzoate. Examples of pyridyl disulfide cross-linkers include 1,4-Di-[3'-2'-pyridyldithio(propionamido)butane] and N-succinimidyl-3-(2-pyridyldithio)-propionate.

Carboxyl groups are cross-linked to primary amines or hydrazides by using carbodimides which result in formation of amide or hydrazone bonds. In this manner, carboxy-termini of peptides or proteins can be linked. Examples of carbodiimide cross-linkers include 1-ethyl-3-(3-dimethylaminopropyl)-carbodiimide hydrochloride and N, N¹-dicyclohexylcarbodiimide. Arylazide cross-linkers become reactive when exposed to ultraviolet radiation and form aryl nitrene. Examples of arylazide cross-linkers include azidobenzoyl hydrazide and N-5-azido-2 nitrobenzoyloxysuccinimide. Glyoxal cross linkers target the guanidyl portion of arginine. An example of a glyoxal cross-linker is p-azidophenyl glyoxal monohydrate.

Heterobifunctional cross-linkers which possess two or more different reactive groups are suitable for use herein. Examples include cross-linkers which are amine-reactive at one end and sulfhydryl-reactive at the other end such as 4-succinimidyl-oxycarbonyl-a-(2-pyridyldithio)-toluene, N- succinimidyl-3-(2-pyridyldithio)-propionate and the maleimide cross-linkers discussed above.

Both surface coupling, as well as bulk coupling within the three-dimensional architecture of hydrogels can be readily obtained with the above-described conjugating chemistry. Indeed, in embodiments, by manipulation of surface and bulk coupling, materials having one type of extracellular matrix adhesion peptide coupled internally in the matrix and another type of extracellular matrix adhesion peptide coupled on the surface can be provided.

In some embodiments extracellular matrix adhesion peptides may be incorporated into cross-linked collagen via amine conjugation using, e.g., the techniques described above, to free amine groups in collagen. For example, terminal carboxylic acid residues on the peptides may be attached to amino groups on collagen using 1-ethy-3-(3-dimethylaminopropyl)-carbodiimide (EDC) and N-hydroxysuccinamide (NHS) chemistry. See, e.g., Steffens et al., Tissue Eng. 2004 Sep-Oct; 10(9-10):1502-9. As another example, cysteine terminated RGD peptides may be attached to amino groups on type 1 collagen via succinimidyl 6-(3-[2-pyridyldithio]-propionamido) hexanoate (Sulfo-LC-SPDP). See, e.g., Burgess et al., Ann Biomed Eng, 2000 Jan:28(1):110-8.

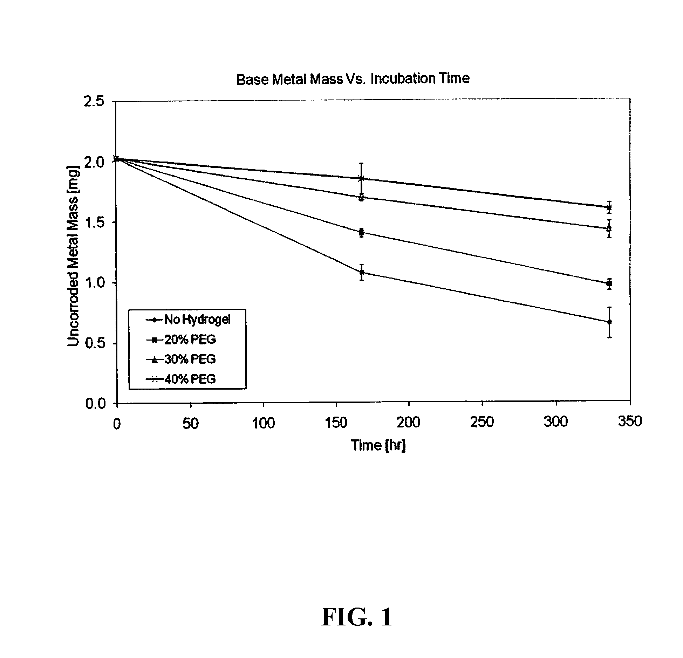

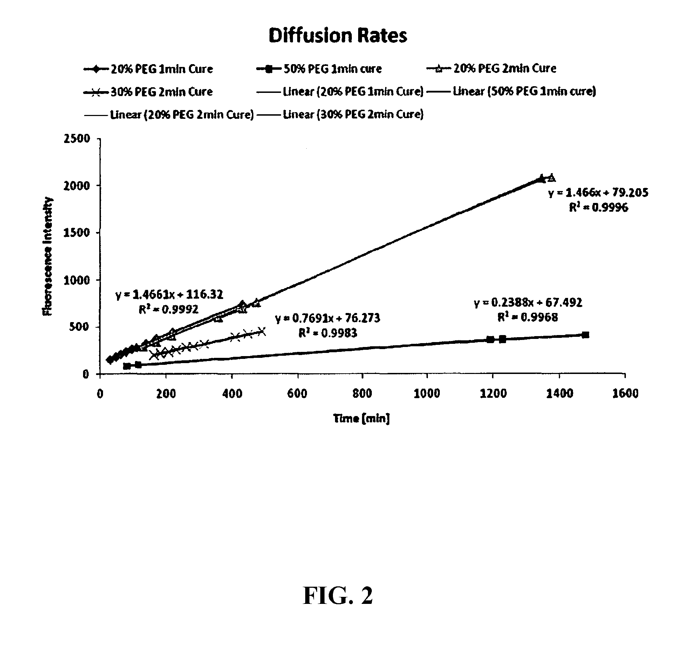

Permeability of the hydrogel may be varied to alter diffusivity of water and ions around the body of the magnesium implant. For example, varying the amount of hydrogel polymeric phase to the amount of aqueous phase affects permeability. In embodiments, the ratio of polymer to aqueous solution may range from about 20% hydrogel polymer and about 80% aqueous solution to about 70% hydrogel polymer and about 30% aqueous solution. Any amount within this range is contemplated, e.g., about 20% polymer, about 30% polymer, about 40% polymer versus a corresponding amount of aqueous phase. As can be seen from FIG. 1, the magnesium degradation rate decreased with increasing PEGDA content. FIG. 2 illustrates diffusion rates of water soluble fluoroceine dye through hydrogel membranes of varying PEGDA content and indicates that diffusivity decreased with increasing PEGDA content. Taken together, it is shown that decreasing permeability and diffusivity to aqueous solution results in slowing of magnesium degradation which may be varied by controlling concentration properties of the hydrogel coating.

The coating may be applied to the implant body by a variety of techniques. The outer surface of the body may be initially treated by etching through exposure to plasma or an acidic solution such as dilute nital solution (1-10 ml nitric acid plus 100 ml ethanol). See, e.g., Zhao et al., Corrosion Science, 2008, 50(7):1939-1953 (3% nital). Other examples include picric acid, e.g., 5gm picric acid plus 0.5 ml acetic acid plus 5ml water plus 25ml ethanol (Zhang et al., Materials Science and Engineering A 2008, 488(1-2):102-111), or 3.5gm picric acid plus 6.5ml acetic acid plus 20ml water plus 100 ml ethanol (Kannan et al., Biomaterials, 2008 May, 29(15):2306-14).

Preparation may include cleaning the outer surface of the base material with a cleaning agent such as isopropyl alcohol or acetone. After the body surface has been etched, one or more adhesion promoting layers made of, e.g., a silane may be applied to the body. In embodiments, initial treatment may include cleaning the outer surface of the base material with isopropyl alcohol, plasma etching the outer surface of the base material and applying the silane to the plasma etched surface. NaOH can be used as a passivating agent to convert Mg to Mg(OH)₂. For example, the body may be washed, e.g., with a 1% NaOH solution, thoroughly rinsed with distilled water and then applying the silane to the NaOH treated surface. Those skilled in the art may determine other suitable concentrations of NaOH. With grit blasting, the outer surface of the base material is grit blasted and then cleaned with isopropyl alcohol and then silane is applied to the cleansed grit blasted surface.

The silane coating may incorporate acrylate or amine terminated functionality through plasma assisted polymerization or solution phase polymerization. The silane provided may have functionality capable of reacting with a nucleophilic group, e.g., a hydroxyl or amino group. In particular, the silane may comprise isocyanate, isothiocyanate, ester, anhydride, acyl halide, alkyl halide, epoxide, or aziridine functionality. In embodiments, the adhesion promoting layer is a thin layer of silane having a thickness in the range of, for example, about 0.5 to about 5,000 Å and preferably, about 2 to about 50 Å. For example, a full monolayer of amine terminated silane (3-aminopropyltrimethoxysilane [APTMS]) may have a thickness of about 10.5 Å. See, e.g., Cui et al., Surface and Interfaces Analysis. 2010. As another example, a full layer of acrylate terminated silane (3-acryloxypropyl) trimethoxysilane (APTS) has a thickness of about 12.5 Å. See, e.g., Müllner et al., J Am Chem Soc. 2010 Nov 24;132(46):16587-92.

After the adhesion promoting layer is applied, the hydrogel layer may be coupled to the adhesion promoting layer via a photoinitiator and UV light or by using crosslinkers such as those described above to link the hydrogel species to silane groups. For example, in embodiments, a photoinitiator is adsorbed to silane, the magnesium body is dip coated in hydrogel monomer solution and then cured through interfacial photopolymerization. In this manner, polymerization occurs close to the magnesium body surface where the photoinitiator is concentrated. For example, 10 µl of 50/mg/ml 2,2-dimethoxy-2-phenyl-acetophenone in dimethyl sulfoxide (DMSO):1 ml PEGDA solution is utilized and cured with UV light for 60 seconds. Excess monomer is rinsed off after polymerization is completed. Alginates may be polymerized through the use of counterions such as Ca⁺⁺.

In embodiments, hydrogel monomers may be applied, e.g., by vapor deposition or plasma deposition, and may polymerize and cure upon condensation from the vapor phase. Plasma is an ionized gas maintained under vacuum and excited by electrical energy, typically in the radiofrequency range. Because the gas is maintained under vacuum, the plasma deposition process occurs at or near room temperature. Plasma can be used to deposit hydrogel polymers onto the adhesion promoting layer. As mentioned above, other coating techniques may be utilized, e.g., dip coating, spray coating, painting or wiping, and the like.

In embodiments, one or more medicinal agents are associated with the hydrogel coating. "Medicinal agent" is used herein its broadest sense and includes any substance or mixture of substances which may have any clinical use. It is to be understood that medicinal agent encompasses any drug, including hormones, antibodies, therapeutic peptides, etc., or a diagnostic agent such as a releasable dye which has no biological activity per se. Growth factors, angiogenic factors and other protein based therapeutic agents can be incorporated into the hydrogel in the same manner as the extracellular matrix adhesion peptides described above which can further encourage cell ingrowth and tissue generation.

Examples of medicinal agents that can be used include anticancer agents, analgesics, anesthetics, anti-inflammatory agents, growth factors such as bone morphogenic proteins (BMPs), antimicrobials, and radiopaque materials. Such medicinal agents are well-known to those skilled in the art. The medicinal agents may be in the form of dry substance in aqueous solution, in alcoholic solution or particles, microcrystals, microspheres or liposomes. An extensive recitation of various medicinal agents is disclosed in Goodman and Gilman, The Pharmacological Basis of Therapeutics, 10th ed. 2001, or Remington, The Science and Practice of Pharmacy, 21 ed. (2005). As used herein, the term "antimicrobial" is meant to encompass any pharmaceutically acceptable agent which is substantially toxic to a pathogen. Accordingly, "antimicrobial" includes antiseptics, antibacterials, antibiotics, antivirals, antifungals and the like. Radiopaque materials include releasable and non-releasable agents which render the implant visible in any known imaging technique such as X-ray radiographs, magnetic resonance imaging, computer assisted tomography and the like. The radiopaque material may be any conventional radiopaque material known in the art for allowing radiographic visualization the implant.

Although the present disclosure has been described with respect to preferred embodiments, it will be readily apparent, to those having ordinary skill in the art that changes and modifications may be made thereto without departing from the spirit or scope of the subject implant.

The invention may be described by reference to the following numbered paragraphs:-
1. A surgical implant comprising a body and a coating over at least a portion of the body, the body including metallic magnesium, the coating including a hydrogel having an extracellular adhesion peptide contained therein.
2. The surgical implant according to paragraph 1 wherein the metallic magnesium is a magnesium alloy.
3. The surgical implant according to paragraph 1 wherein the hydrogel is non-degradable.
4. The surgical implant according to paragraph 1 wherein the hydrogel is degradable.
5. The surgical implant according to paragraph 1 wherein the hydrogel is selected from the group consisting of polyethylene glycol, alginate, collagen and polyurethane.
6. The surgical implant according to paragraph 5 wherein the polyethylene glycol is polyethylene glycol acrylate.
7. The surgical implant according to paragraph 6 wherein the polyethylene glycol acrylate is selected from the group consisting of polyethylene glycol diacrylate, polyethylene glycol dimethacrylate and multiarm polyethylene glycol acrylate.
8. The surgical implant according to paragraph 1 wherein the extracellular matrix adhesion peptide is covalently bonded to the hydrogel.
9. The surgical implant according to paragraph 1 wherein the extracellular matrix adhesion peptide is selected from the group consisting of RGD, YIGSR, KQAGDV, REDV, PHSRN, IKVAV, PDGSR, LRGDN, LRE, IKLLI, GFOGER and VAPG.
10. The surgical implant according to paragraph 1 further comprising a medicinal agent.
11. The surgical implant according to paragraph 1 further comprising an adhesion promoting layer between the body and the hydrogel.
12. The surgical implant according to paragraph 11 wherein the adhesion promoting layer comprises a silane.
13. The surgical implant according to paragraph 1 wherein the hydrogel includes from about 20% to about 70% of a polymeric phase and from about 80% to about 30% of an aqueous phase.
14. The surgical implant according to paragraph 1 wherein the hydrogel coating encapsulates the entire implant
15. A method of making a surgical implant comprising
   providing a magnesium based degradable implant body;
   applying and adhering a functionalized reactive silane based adhesion promoting layer to the implant body;
   providing a hydrogel monomeric solution having extracellular matrix adhesion peptides incorporated therein; and
   contacting the hydrogel monomeric solution with the adhesion promoting layer such that the hydrogel polymerizes and bonds to the adhesion promoting layer and encapsulates at least a portion of the implant.
16. The method of making a surgical implant according to paragraph 15 wherein the silane is functionalized with a heterobifunctional crosslinker or a homobifunctional crosslinker.
17. The method of making a surgical implant according to paragraph 15 wherein the hydrogel is selected from the group consisting of polyethylene glycol, alginate, collagen and polyurethane.
18. The method of making a surgical implant according to paragraph 15 wherein the extracellular matrix adhesion peptides are selected from the group consisting of RGD, YIGSR, KQAGDV, REDV, PHSRN, IKVAV, PDGSR, LRGDN, LRE, IKLLI, GFOGER and VAPG.
19. The method of making a surgical implant according to paragraph 15 wherein the extracellular matrix adhesion peptides are covalently bonded to the hydrogel.
20. The method of making a surgical implant according to paragraph 15 wherein the hydrogel coating encapsulates the entire implant.
21. The method of making a surgical implant according to paragraph 15 further comprising etching the implant body prior to applying and adhering a functionalized reactive silane based adhesion promoting layer to the implant body.

## Claims

1. A surgical implant comprising a body and a coating over at least a portion of the body, the body including metallic magnesium, the coating including a hydrogel having an extracellular adhesion peptide contained therein.

2. The surgical implant according to claim 1 wherein the metallic magnesium is a magnesium alloy.

3. The surgical implant according to claim 1 or claim 2, wherein the hydrogel is selected from the group consisting of polyethylene glycol, alginate, collagen and polyurethane.

4. The surgical implant according to claim 3 wherein the polyethylene glycol is polyethylene glycol acrylate, optionally selected from the group consisting of polyethylene glycol diacrylate, polyethylene glycol dimethacrylate and multiarm polyethylene glycol acrylate.

5. The surgical implant according to any preceding claim, further comprising a medicinal agent.

6. The surgical implant according to any preceding claim, further comprising an adhesion promoting layer between the body and the hydrogel, wherein the adhesion promoting layer optionally comprises a silane.

7. The surgical implant according to any preceding claim, wherein the hydrogel includes from about 20% to about 70% of a polymeric phase and from about 80% to about 30% of an aqueous phase.

8. The surgical implant according to any preceding claim, wherein the hydrogel coating encapsulates the entire implant.

9. A method of making a surgical implant comprising
providing a magnesium based degradable implant body;
applying and adhering a functionalized reactive silane based adhesion promoting layer to the implant body;
providing a hydrogel monomeric solution having extracellular matrix adhesion peptides incorporated therein; and
contacting the hydrogel monomeric solution with the adhesion promoting layer such that the hydrogel polymerizes and bonds to the adhesion promoting layer and encapsulates at least a portion of the implant.

10. The method of making a surgical implant according to claim 9 wherein the silane is functionalized with a heterobifunctional crosslinker or a homobifunctional crosslinker.

11. The method of making a surgical implant according to claim 9 or claim 10, wherein the hydrogel is selected from the group consisting of polyethylene glycol, alginate, collagen and polyurethane.

12. The surgical implant according to any one of claims 1 to 8, or the method of making a surgical implant according to any one of claims 9 to 11, wherein the extracellular matrix adhesion peptides are selected from the group consisting of RGD, YIGSR, KQAGDV, REDV, PHSRN, IKVAV, PDGSR, LRGDN, LRE, IKLLI, GFOGER and VAPG.

13. The surgical implant according to any one of claims 1 to 8, or the method of making a surgical implant according to any one of claims 9 to 12, wherein the extracellular matrix adhesion peptides are covalently bonded to the hydrogel.

14. The method of making a surgical implant according one of claims 9 to 13 wherein the hydrogel coating encapsulates the entire implant.

15. The method of making a surgical implant according to one of claims 9 to 14 further comprising etching the implant body prior to applying and adhering a functionalized reactive silane based adhesion promoting layer to the implant body.
